# EUROPÄISCHE PATENTSCHRIFT

(11) **EP 3 238 637 B1**
(45) Veröffentlichungstag und Bekanntmachung des Hinweises auf die Patenterteilung: **05.09.2018**
(21) Anmeldenummer: 17162578.3
(22) Anmeldetag: 13.11.2009
(51) Int. Cl.: A61B 17/00, A61B 17/068, A61B 17/32, A61B 90/00

(54) **DRUCKGASBETRIEBENES INSTRUMENT, INSBESONDERE CHIRURGISCHES INSTRUMENT**
COMPRESSED GAS OPERATED INSTRUMENT, IN PARTICULAR SURGICAL INSTRUMENT
INSTRUMENT ENTRAÎNÉ PAR GAZ SOUS PRESSION, EN PARTICULIER INSTRUMENT CHIRURGICAL

(30) Priorität: 17.07.2009 DE 102009033525
(43) Veröffentlichungstag der Anmeldung: 01.11.2017
(62) Teilanmeldung aus: 09751909.4
(73) Patentinhaber: Aesculap AG, 78532 Tuttlingen (DE)
(72) Erfinder: SCHOLTEN, Thomas, 78532 Tuttlingen (DE); MAYENBERGER, Rupert, 78239 Rielasingen (DE)
(74) Vertreter: Hoeger, Stellrecht & Partner Patentanwälte mbB

(56) Entgegenhaltungen:
- DE-B3-102006 024 759
- DE-U1-202006 008 404
- DE-U1-202007 006 801
- US-A- 3 815 476
- US-A- 4 709 697
- US-A1- 2005 238 555
- US-A1- 2006 069 395
- US-A1- 2006 112 944

## Beschreibung

Die Erfindung betrifft ein druckgasbetriebenes Instrument, insbesondere ein chirurgisches Instrument, mit einem Anschluss für eine Druckgaspatrone, an dem die Druckgaspatrone abgedichtet mit einem Zufuhrkanal für aus der Druckgaspatrone ausströmendes Druckgas verbindbar ist, und mit einem Regelventil in dem Zufuhrkanal, welches den Druckgasstrom durch den Zufuhrkanal beeinflusst.

Ein medizinisches Instrument, welches mittels einer Druckgaspatrone betrieben wird, ist beispielsweise in der DE 20 2007 006 801 U1 beschrieben. Die Verbindung zwischen dem Innenraum der Gaspatrone und dem Zufuhrkanal des Instrumentes wird dabei durch einen Anstechdorn erzielt, durch den das Druckgas aus der Druckgaspatrone in den Zufuhrkanal einströmt. Da der Druck in der Druckgaspatrone normalerweise erheblich höher ist als der Druck, der zum Betrieb des Instrumentes benötigt wird, ist eine Druckminderung notwendig, und dies kann mit Hilfe eines Regelventils erfolgen, das in den Zufuhrkanal eingeschaltet wird.

Die US 2005/0238555 A1 beschreibt einen Gasregler mit einem Gehäuse und einem Druckregler im Gehäuse längs einer ersten Achse zum Regeln des Druckes eines Gases, das von einer Druckquelle bereitgestellt wird. Ein Durchflussmesser regelt die Flussrate des vom Druckregler erhaltenen Gasdruckes. Der Flussmesser ist relativ zum Gehäuse längs einer zweiten Achse positioniert, die quer zur ersten Achse ausgerichtet ist. Eine Auslassdüse hat einen Düsenkörper und ist längs der ersten Achse ausgerichtet. Die Auslassdüse empfängt Gas vom Flussmesser und gibt Gas durch den Düsenkörper ab.

Druckgasbetriebene Instrumente für medizinische Anwendungen sind in der DE 10 2006 024 759 B3, der DE 20 2006 008 404 U1, der DE 20 2007 006 801 U1, der US 3,815,476, der US 2006/0069395 A1 und in der US 4,709,697 beschrieben.

Es ist Aufgabe der Erfindung, ein druckgasbetriebenes Instrument so auszubilden, dass mit einfachen Mitteln und mit geringem Platzbedarf eine Regelung des Gasdruckes erfolgen kann, der für den Betrieb des Instrumentes notwendig ist.

Diese Aufgabe wird erfindungsgemäß durch ein druckgasbetriebenes Instrument mit den Merkmalen von Anspruch 1 gelöst.

Die erfindungsgemäße Ausgestaltung führt dazu, dass zunächst beim Anschluss einer Druckgaspatrone Druckgas durch den Zufuhrkanal und durch die Ventilkammer hindurchströmen kann, da der Ventilkörper durch das Federelement in der Öffnungsstellung gehalten ist. Sobald jedoch der Druck ansteigt, wird die Druckdifferenz, die auf den Ventilkörper wirkt, größer, da die von der zweiten Dichtung umschlossene Querschnittsfläche kleiner ist als die von der ersten Dichtung umschlossene Querschnittsfläche. Beim Überschreiten eines bestimmten Wertes ist die Differenzkraft, die durch das Druckgas auf den Ventilkörper wirkt, größer als die Kraft des Federelementes, die den Ventilkörper in die Öffnungsstellung verschiebt, und dann wird der Ventilkörper in die Schließstellung verschoben und verschließt auf diese Weise den Strömungskanal in dem Anschlusselement. Ein weiterer Druckanstieg ist damit stromabwärts des Ventilkörpers nicht mehr möglich, sondern der Druck wird in diesem Bereich durch Abströmen des Gases in das Instrument weiter abgebaut, bis die Kraft des Federelementes wieder die Differenzkraft des Druckgases auf den Ventilkörper übersteigt und dadurch den Ventilkörper wieder in die Öffnungsstellung verschiebt.

Auf diese Weise wird mit einfachen Mitteln eine automatische Druckbegrenzung erreicht, sobald der Druck des Druckgases im Zufuhrkanal einen bestimmten Wert überschreitet, wird der Zufuhrkanal gesperrt, wenn der Druck wieder unter einen bestimmten Wert fällt, erfolgt eine neue Öffnung und eine Nachlieferung von Druckgas aus der Druckgaspatrone.

Erfindungsgemäß ist vorgesehen, dass stromabwärts des Regelventils im Zufuhrkanal ein Schließkörper verschieblich gelagert ist, der von einem von der stromabwärts gelegenen Seite in den Zufuhrkanal eingeschobenen Anschlussstutzen von einer stromabwärts gelegenen Schließposition in eine stromaufwärts gelegene Öffnungsposition verschiebbar ist, dass der Schließkörper in der Öffnungsposition den Durchgang durch den Strömungskanal freigibt und ihn in der Schließposition verschließt und dass der Schließkörper einen Durchlass mit sehr kleinem Querschnitt aufweist, der auch bei der Stellung des Schließkörpers in der Schließposition eine sehr reduzierte Strömung des Druckgases an dem Schließkörper vorbei zulässt.

Im normalen Betrieb wird dieser Schließkörper durch den in den Zufuhrkanal eingeschobenen Anschlussstutzen des Instrumentes in die Öffnungsposition verschoben und behindert damit den Durchfluss durch den Zufuhrkanal nicht. Wenn der Anschlussstutzen entfernt ist, gelangt jedoch der Schließkörper in die Schließposition und verschließt den Querschnitt des Zufuhrkanals, allerdings mit Ausnahme eines sehr geringen Querschnittes, durch den in geringem Umfange Druckgas ausströmen kann. Dadurch wird sichergestellt, dass nach der Benutzung des Instrumentes und nach der Entfernung des Anschlussstutzens aus dem Zufuhrkanal eine Druckgaspatrone, die im Instrument verblieben ist, allmählich geleert wird. Dadurch ist der Benutzer gezwungen, bei einer nächsten Benutzung eine neue Druckgaspatrone einzusetzen, also die Benutzung immer mit einer vollen Druckgaspatrone zu beginnen. Da zwischen den einzelnen Benutzungen normalerweise relativ große Zeiträume liegen, kann diese Entleerung der Druckgaspatrone sehr langsam erfolgen, das heißt der freie Querschnitt des Schließkörpers in der Schließposition kann sehr klein sein, beispielsweise kann die Entleerung der Druckgaspatrone über Stunden erfolgen.

Günstig ist es, wenn das Federelement eine Tellerfeder ist, man erhält dadurch hohe Federkräfte bei geringem Platzbedarf.

Bei einer bevorzugten Ausführungsform ist vorgesehen, dass der Ventilkörper eine zentrale, zur Einströmseite hin offene Lagerkammer aufweist, in die ein Lagerstutzen des Anschlusselementes mittels der zweiten Dichtung abgedichtet und verschiebbar eintaucht, und dass aus der Lagerkammer an deren ausströmseitigem Ende der Strömungsdurchlass austritt. Damit ergibt sich eine teleskopierende, abgedichtete Verbindung zwischen dem Lagerstutzen des Anschlusselementes einerseits und dem Ventilkörper andererseits.

Es ist günstig, wenn der Ventilkörper in der Schließstellung an der ausströmseitigen Stirnseite des Anschlusselementes anliegt und dadurch den Strömungskanal verschließt.

Bei einer besonders bevorzugten Ausführungsform ist weiterhin vorgesehen, dass das Anschlusselement durch ein Federelement in eine Dichtstellung gedrückt wird, in der das Anschlusselement dicht an dem in die Ventilkammer eintretenden Zufuhrkanal anliegt und diesen dadurch dicht mit dem Strömungskanal im Anschlusselement verbindet, und gegen die Wirkung des Federelementes von dem Zufuhrkanal in eine Entlastungsstellung verschiebbar ist, in der der in die Ventilkammer eintretende Zufuhrkanal in den stromaufwärts der ersten Dichtung gelegenen Teil der Ventilkammer geöffnet ist. Während in der Dichtstellung die Verbindung zwischen dem Zufuhrkanal einerseits und dem Strömungskanal im Anschlusselement abgedichtet ist, besteht in der Entlastungsstellung für das Druckgas die Möglichkeit, seitlich in die Ventilkammer zu entweichen und dadurch nicht in den Strömungskanal des Anschlusselementes einzutreten, dieser Teil der Ventilkammer ist in der Regel belüftet und steht mit der Umgebung in Verbindung, so dass auf diese Weise eine Druckentlastung erfolgen kann. Die Dimensionierung ist dabei so gewählt, dass eine Verschiebung des Anschlusselementes in die Entlastungsstellung nur dann erfolgt, wenn der Druck in der Druckgaspatrone einen sehr hohen Wert übersteigt. Dies kann der Fall sein, wenn nach der Benutzung des Instrumentes die Druckgaspatrone im Instrument verbleibt und das Instrument zur Sterilisation gegeben wird. Durch die sehr hohen Temperaturen während der Sterilisation können in der Druckgaspatrone Druckwerte entstehen, die weit oberhalb des normalen Speicherdruckes liegen und zu einer Zerstörung der Druckgaspatrone führen könnten. Dies wird durch das verschiebbare Anschlusselement vermieden, das somit als Sicherheitsventil gegen überhöhten Druck in der Druckgaspatrone wirkt.

Besonders vorteilhaft ist es, wenn das den Ventilkörper in die Öffnungsstellung und das Anschlusselement in die Dichtstellung verschiebende Federelement dasselbe Federelement ist. Dieses Federelement schiebt damit den Ventilkörper und das Anschlusselement auseinander in die jeweiligen Endstellungen und wird komprimiert sowohl bei der Verschiebung des Ventilkörpers in die Schließstellung als auch bei der Verschiebung des Anschlusselementes in die Entlastungsstellung. Dadurch ergibt sich eine besonders geringe Bauhöhe der Gesamtanordnung.

Gemäß einer bevorzugten Ausführungsform der Erfindung ist dabei vorgesehen, dass das Verhältnis der stromabwärts gelegenen Querschnittsfläche des Ventilkörpers zu der stromaufwärts gelegenen Querschnittsfläche des Anschlusselements größer ist als das Verhältnis des Druckes in der Druckgaspatrone zu dem geregelten Druck stromabwärts des Ventilkörpers bei Raumtemperatur und kleiner ist als das Verhältnis diese Drücke bei gegenüber Raumtemperatur deutlich erhöhter Temperatur.

Der Durchlass kann ein durch den Schließkörper hindurchgehender Kanal mit geringem Querschnitt sein, also im Wesentlichen eine sehr enge Drosselbohrung, bei einer anderen Ausführungsform kann vorgesehen sein, dass der Durchlass durch einen porösen Wandbereich des Schließkörpers gebildet wird.

Es ist vorteilhaft, wenn der Schließkörper durch eine Feder in Richtung auf die Schließposition belastet ist. Dadurch ist sichergestellt, dass der Schließkörper nach dem Entfernen des Anschlussstutzens immer selbständig in die Schließposition verschoben wird.

Es kann weiterhin vorgesehen sein, dass der Schließkörper in der Schließposition durch eine Dichtung gegenüber dem Zufuhrkanal abgedichtet ist.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Längsschnittansicht eines Anschlusses einer Druckgaspatrone mit eingesetzter Druckgaspatrone;
- Figur 2:: eine Teilansicht des Anschlussteils der Figur 1 in Richtung des Pfeiles A in Figur 1;
- Figur 3:: eine Detailansicht entsprechend dem Ausschnitt B in Figur 2 mit dem Regelventil in Öffnungsstellung;
- Figur 4:: eine Ansicht ähnlich Figur 3 mit dem Regelventil in Schließstellung;
- Figur 5:: eine Ansicht ähnlich Figur 3 mit dem Anschlusselement in Entlastungsstellung;
- Figur 6:: eine Ansicht ähnlich Figur 3 ohne instrumentenseitigen Anschlussstutzen am Ausströmende des Anschlusses und mit teilweise aus dem Anschlussschacht herausgezogener Druckgaspatrone;
- Figur 7:: eine Ansicht des Ausschnittes C in Figur 6 in vergrößerter Darstellung;
- Figur 8:: eine Ansicht ähnlich Figur 7 bei einem abgewandelten Ausführungsbeispiel mit einer Ringdichtung im Dichtstück und einer Erweiterung in der Innenwand des Anschlussschachtes;
- Figur 9:: eine Ansicht ähnlich Figur 8 mit einer aufgeweiteten, in die Erweiterung in der Innenwand des Anschlussschachtes eintretender Ringdichtung;
- Figur 10:: eine Draufsicht auf das Dichtstück der Figur 9 im Bereich der Ringdichtung mit einer seitlichen Ausnehmung der die Ringdichtung aufnehmenden Umfangsnut;
- Figur 11:: eine vergrößerte Schnittansicht des ausströmseitigen Endes des Anschlusses mit dem Schließkörper in Schließposition;
- Figur 12:: eine Schnittansicht der Druckgaspatrone mit der Druckgaspatrone in der richtigen Einbaurichtung und

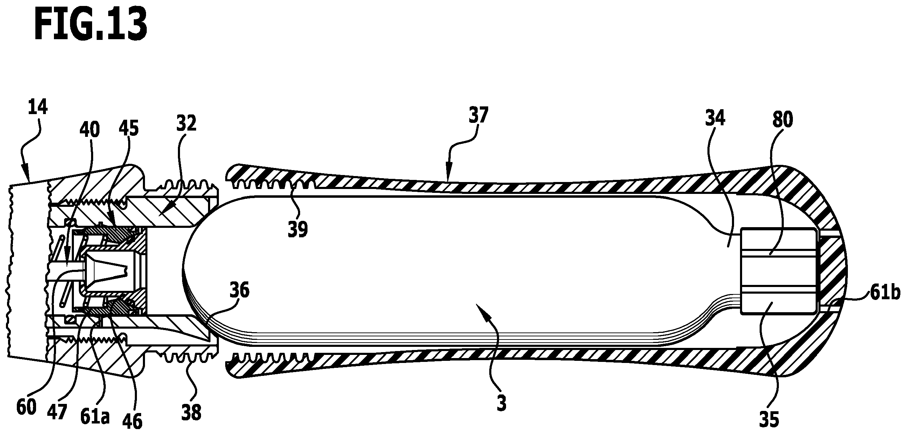

- Figur 13:: eine Schnittansicht ähnlich Figur 9 mit der Druckgaspatrone in falscher Einbaurichtung.

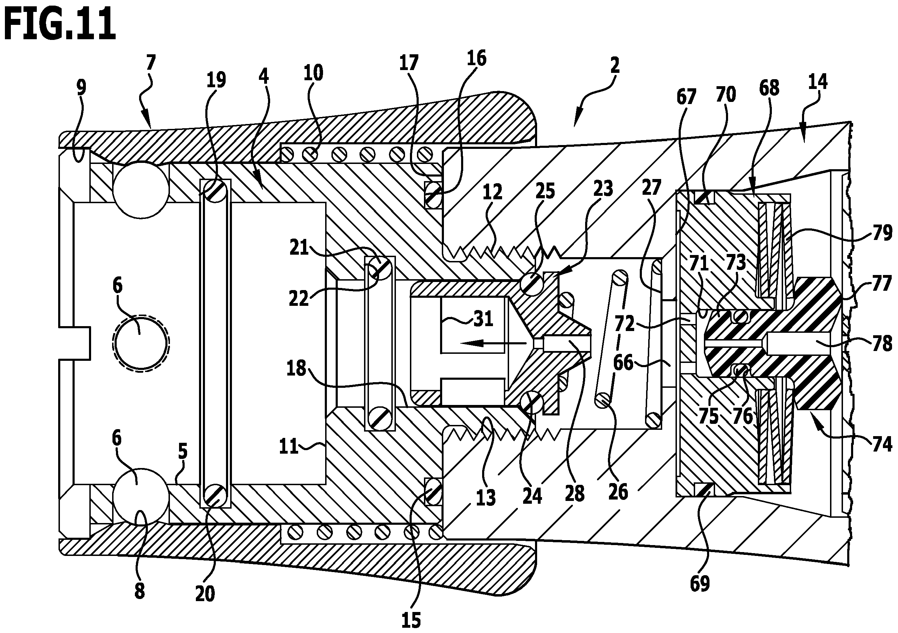

In der Zeichnung ist eine Druckgasversorgung für ein Instrument dargestellt, insbesondere ein medizinisches Instrument, beispielsweise ein Instrument zum Setzen von Heftklammern, zum Antrieb eines Schneidgerätes oder dergleichen. Von diesem Instrument ist jedoch in der Zeichnung nur ein rohrförmiger Anschlussstutzen 1 dargestellt, der über einen Anschluss 2 mit einer Druckgaspatrone 3 verbunden wird. Nachfolgend wird dieser Anschluss näher beschrieben, es versteht sich aber, dass dieser Anschluss über einen Anschlussstutzen 1 mit einem beliebigen druckgasbetriebenen Instrument verbunden werden kann. Zu diesem Zweck weist der Anschluss eine Einsteckbuchse 4 auf mit einer zylindrischen Einsteckkammer 5, in diese Einsteckkammer 5 kann von einer Seite der ebenfalls zylindrische Anschlussstutzen 1 eingeschoben werden und dort mit Hilfe von in der Einsteckbuchse 4 radial verschieblich gelagerten kugelförmigen Sperrelementen 6 in axialer Richtung fixiert werden. Diese Sperrelemente 6 können zwischen einer radial eingeschobenen Stellung, in der sie in entsprechende Vertiefungen des Anschlussstutzens 1 eintauchen, und einer radial ausgeschobenen Freigabestellung verschoben werden, in der sie so weit nach außen verlagert sind, dass der Anschlussstutzen 1 frei in die Einsteckkammer 5 eingeschoben und wieder aus dieser herausgezogen werden kann. Zur Festlegung der kugelförmigen Sperrelemente 6 in der eingeschobenen Sperrstellung dient eine die Einsteckbuchse 4 umgebende und gegenüber dieser axial verschiebliche Hülse 7 mit zwei nebeneinander angeordneten Abschnitten 8, 9, die jeweils unterschiedliche Innendurchmesser aufweisen. Bei Anlage des Abschnittes 8 mit kleinem Innendurchmesser werden die kugelförmigen Sperrelemente 6 radial nach innen geschoben, bei Anlage des Abschnittes 9 mit dem größeren Innendurchmesser können die kugelförmigen Sperrelemente 6 radial nach außen verschoben werden. Die Hülse 7 wird durch eine die Einsteckbuchse 4 umgebende Schraubenfeder 10 so verschoben, dass der Abschnitt 8 mit kleinerem Innendurchmesser die kugelförmigen Sperrelemente 6 überdeckt. Um diese kugelförmigen Sperrelemente 6 freigeben zu können, muss die Hülse 7 entgegen der Wirkung der Schraubenfeder 10 radial zurückgeschoben werden (Figur 11).

An ihrem dem offenen Ende der Einsteckkammer 5 gegenüberliegenden Ende trägt die Einsteckbuchse 4 einen zentralen Außengewindestutzen 12, mit dem die Einsteckbuchse 4 in eine Innengewindebohrung 13 eines zentralen Anschlussstückes 14 eingeschraubt ist. Dabei wird die Einsteckbuchse 4 gegenüber dem zentralen Anschlussstück 14 durch eine Ringdichtung 15 abgedichtet, die in einer Ringnut 16 an der Rückseite der Einsteckbuchse 4 angeordnet ist und sich an der Stirnseite 17 des zentralen Anschlussstückes 14 anlegt.

Die Einsteckkammer 5 geht unter Ausbildung einer Stufe 11 über in einen zentralen Strömungskanal 18, der den Außengewindestutzen 12 zentral durchsetzt. In eine Umfangsnut 19 an der Innenwand der Einsteckkammer 5 ist eine Ringdichtung 20 eingelegt, eine weitere Ringdichtung 21 ist in eine Umfangsnut 22 in der Innenwand des zentralen Strömungskanals 18 eingelegt.

In den zentralen Strömungskanal 18 ist in Längsrichtung des Strömungskanals 18 verschiebbar ein Schließkörper 23 eingesteckt, der teilweise aus dem Außengewindestutzen 12 hervorsteht und dort einen Außendurchmesser aufweist, der größer ist als der Innendurchmesser des zentralen Strömungskanals 18. In diesem Bereich ist in eine Umfangsnut 24 des Schließkörpers 23 eine Ringdichtung 25 eingelegt, die sich an die Außenkante des zentralen Strömungskanals 18 anlegt, wenn man den Schließkörper in Richtung auf die Einsteckkammer 5 verschiebt. In dieser Stellung verschließt der Schließkörper 23 damit den Strömungskanal 18, diese Stellung wird als Schließposition bezeichnet. In diese Position wird der Schließkörper durch eine Schraubenfeder 26 gedrückt, die sich einerseits an der Außenseite des Schließkörpers 23 und andererseits an einer Stufe 27 des Anschlussstückes 14 abstützt.

In dem Schließkörper 23 ist ein Durchlass 28 in Form einer Bohrung mit sehr kleinem Durchmesser angeordnet. Dieser Durchlass 28 verbindet den Innenraum des Anschlussstückes 14 damit mit dem zentralen Strömungskanal 18. Der Durchmesser ist jedoch so gering, dass pro Zeiteinheit nur eine sehr geringe Gasmenge hindurchtreten kann, in der Zeichnung ist der Durchmesser dieses Durchlasses 28 zur Verdeutlichung relativ groß ausgebildet. Tatsächlich kann es sich dabei um eine ganz dünne Bohrung handeln oder auch um einen porösen Wandabschnitt an der Rückseite des Schließkörpers, der einen langsamen Abbau eines Gasüberdruckes aus dem Inneren des Anschlussstückes 14 in Richtung auf die offene Einsteckkammer 5 hin zulässt.

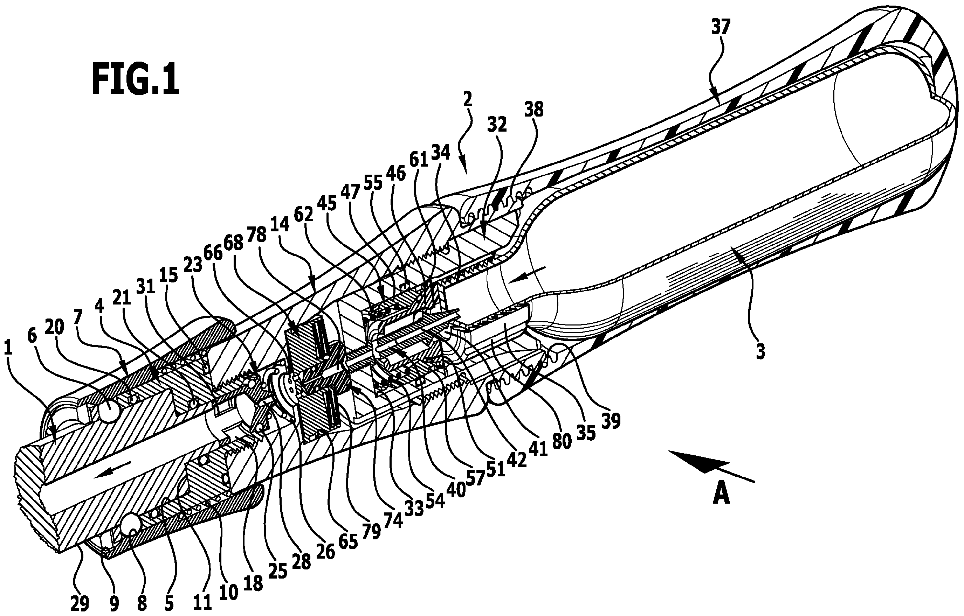

Der Anschlussstutzen 1 eines Instrumentes weist einen ersten Abschnitt 29 mit einem Außendurchmesser auf, der dem Innendurchmesser der Einsteckbuchse 4 entspricht, und einen zweiten Abschnitt 30 mit einem wesentlich geringeren Außendurchmesser, der dem Innendurchmesser des zentralen Strömungskanals 18 entspricht (Figur 1). Beim Einsetzen des Anschlussstutzens 1 in die Einsteckbuchse 4 wird der Abschnitt 29 in der Einsteckbuchse 4 aufgenommen, der Abschnitt 30 dagegen in dem zentralen Strömungskanal 18. Dabei schiebt der Abschnitt 30 des Anschlussstutzens 1 den Schließkörper 23 entgegen der Wirkung der Schraubenfeder 26 aus dem zentralen Strömungskanal 18 heraus in eine Öffnungsposition, in der die Ringdichtung 25 von der Kante des zentralen Strömungskanals 18 abgehoben ist, so dass die Schließwirkung in diesem Bereich aufgehoben ist. In der Seitenwand des Schließkörpers 23 sind Fensteröffnungen 31 angeordnet, die eine Verbindung zwischen dem Innenraum des Anschlussstutzens 1 einerseits und dem Innenraum des Anschlussstückes 14 andererseits herstellen, sobald der Schließkörper 23 in der Öffnungsposition steht. In dieser Position wird also ein Gasstrom aus dem Innenraum des Anschlussstückes 14 in den Innenraum des Anschlussstutzens 1 nicht behindert.

Eine Behinderung tritt erst auf, wenn der Schließkörper wieder in der Schließposition steht, und dies setzt voraus, dass der Anschlussstutzen 1 aus der Einsteckbuchse 4 entfernt ist, wie dies in Figur 11 dargestellt ist. Nur in dieser Stellung wird der Durchlass 28 überhaupt wirksam. In der Öffnungsstellung ist der Querschnitt des Durchlasses 28 gegenüber den übrigen Strömungsquerschnitten für den Gasstrom so klein, dass er praktisch nicht ins Gewicht fällt. In der Schließposition dagegen erfolgt infolge des Durchlasses 28 kein hermetisches Verschließen, sondern der Druck im Inneren des Anschlussstückes 14 kann sich über diesen Durchlass 28 allmählich abbauen, wobei die Dauer dieses Abbaus vom Querschnitt des Durchlasses 28 abhängt.

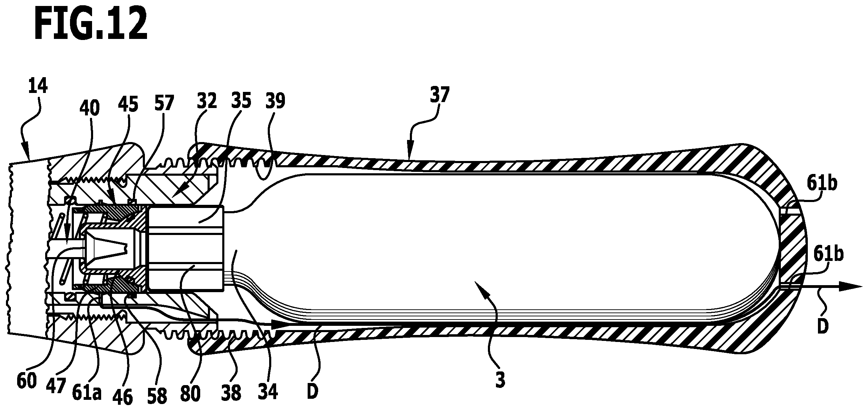

Auf der der Einsteckbuchse 4 gegenüberliegenden Seite des Anschlussstückes 14 ist in dieses ein topfförmiger Aufnahmekörper 32 eingeschraubt. Am hinteren Ende des Anschlussstückes 14 bildet ein offener Innenraum des Aufnahmekörpers 32 einen Anschlussschacht 33 für den zylindrischen Anschlussbereich 34 der Druckgaspatrone 3. Diese zylindrische Druckgaspatrone 3 ist am rückwärtigen Ende kugelförmig abgerundet, am vorderen Ende läuft sie flaschenhalsähnlich aus in einem zylindrischen Anschlussbereich 34, der von einer Dichtkappe 35 aus einem verformbaren Kunststoffmaterial überfangen ist. Die Druckgaspatrone 3 kann mit dem Anschlussbereich 34 in den Anschlussschacht 33 eingeschoben werden, die Einschubtiefe wird begrenzt dadurch, dass der Bereich der Druckgaspatrone 3 mit größerem Außendurchmesser am entsprechend geformten Rand 36 des Aufnahmekörpers 32 anschlägt. Dieser Rand 36 verhindert im Übrigen auch, dass die Druckgaspatrone 3 verkehrt in den Anschlussschacht 33 eingesetzt wird, also mit dem kugelförmigen Ende voran, wie dies in Figur 12 dargestellt ist. In diesem Falle schlägt das kugelförmige Ende an dem Rand 36 an und verhindert somit eine weitere Annäherung der Druckgaspatrone an das Anschlussstück 14 und damit auch eine Beschädigung des Anstechdornes 40 (siehe unten).

Die Druckgaspatrone 3 kann mittels einer Überwurfhülse 37 an dem Anschlussstück 14 festgelegt werden. Dazu trägt das Anschlussstück 14 an seinem rückwärtigen Ende ein Außengewinde 38, auf welches die Überwurfhülse 37 mit einem Innengewinde 39 aufgeschraubt werden kann. Die Dimensionierung ist dabei so gewählt, dass die Überwurfhülse 37 die Druckgaspatrone 3 bei korrekter Orientierung so weit gegen das Anschlussstück 14 verschiebt, bis der Bereich mit größerem Außendurchmesser der Druckgaspatrone 3 an dem Rand 36 des Aufnahmekörpers 32 anliegt. Ist die Druckgaspatrone falsch orientiert, steht sie so weit von dem Anschlussstück 14 vor, dass die über die falsch orientierte Druckgaspatrone 3 geschobene Überwurfhülse mit ihrem Innengewinde 39 das Außengewinde 38 nicht erreichen kann. Ein Aufschrauben der Überwurfhülse 37 ist damit nicht möglich. Dies dient der Kontrolle für die korrekte Orientierung der Druckgaspatrone 3 relativ zu dem Anschlussstück 14.

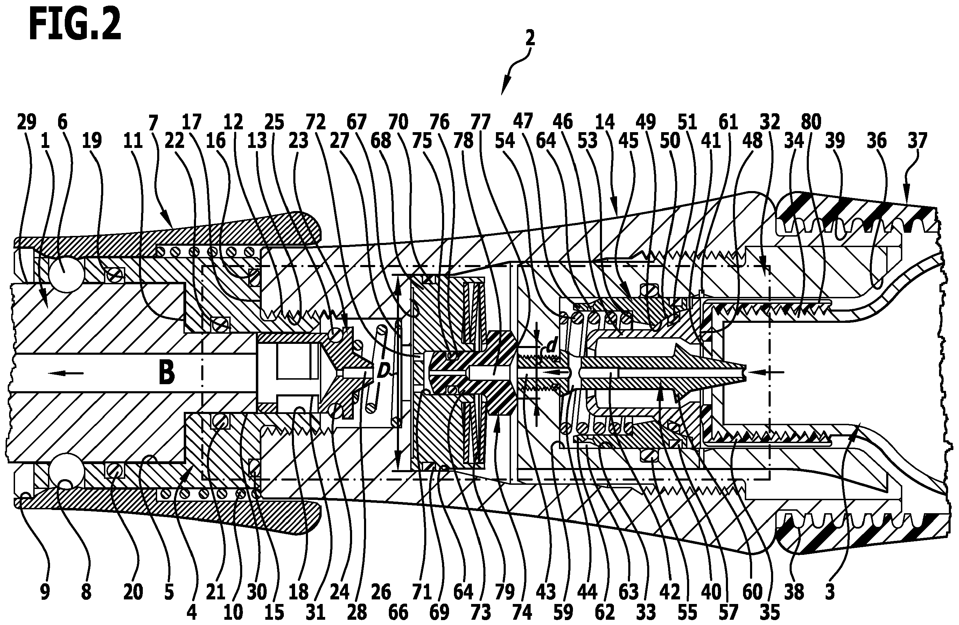

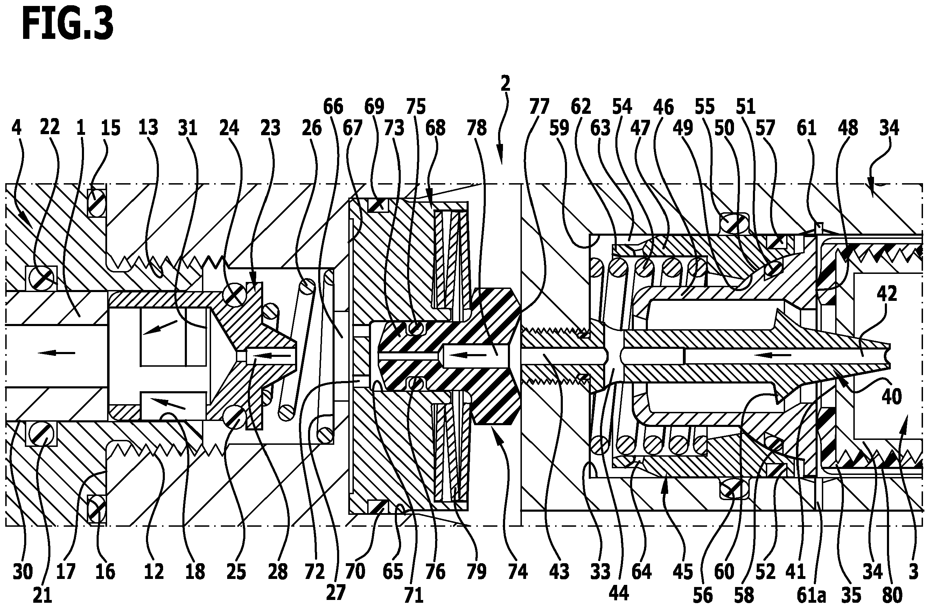

In dem Anschlussschacht 33 ist ein zentraler Anstechdorn 40 angeordnet, der gegenüber dem Aufnahmekörper 32 in axialer Richtung unverschieblich gelagert ist, beispielsweise ist er in den Boden des Anschlussschachtes eingeschraubt. Dieser Anstechdorn 40 steht vom Boden des Anschlussschachtes 33 so weit hervor, dass er die Druckgaspatrone 3 an ihrer Stirnfläche 41 durchbohrt, wenn die Druckgaspatrone 3 vollständig in den Anschlussschacht 33 eingeschoben ist (Figuren 2 und 3).

Der Anstechdorn 40 wird von einem rohrförmigen Kanal 42 durchsetzt, der ein Teil des gesamten Strömungskanals vom Inneren der Druckgaspatrone 3 bis zu dem Anschlussstutzen 1 ist, also ein Teil eines das Anschlussstück 14 durchsetzenden Zufuhrkanals 43 für das Druckgas. Der Kanal 42 in dem Anstechdorn 40 steht außerdem über eine Querbohrung 44 mit dem Innenraum des Anschlussschachtes 33 in Verbindung.

In dem Anschlussschacht 33 ist den Anstechdorn 40 im Abstand umgebend ein im Anschlussschacht 33 in dessen Längsrichtung verschieblich gelagertes Dichtstück 45 angeordnet, welches aus zwei Teilen besteht, nämlich einem hutförmigen Innenteil 46 und einem hülsenförmigen Außenteil 47. Das Innenteil 46 weist an seinem der Druckgaspatrone 3 zugewandten Ende eine stirnseitige Dichtfläche 48 auf, die sich bei eingeschobener Druckgaspatrone 3 an deren Dichtkappe 35 dichtend anlegt, diese Dichtkappe 35 kann zur Verbesserung der Dichtwirkung im Dichtbereich wulstförmig verbreitert sein.

Der hülsenförmige Außenteil 47 stützt sich mit einer kugelkalottenförmigen Innenfläche 49 an einer ebenfalls kugelkalottenförmigen Außenfläche 50 des Innenteils 46 ab, in diesem Bereich sind Innenteil 46 und Außenteil 47 durch eine Ringdichtung 51 gegeneinander abgedichtet, die in eine Umfangsnut 52 in der Außenfläche 50 eingelegt ist. An einer Stufe 53 des Außenteils 47 stützt sich eine den Anstechdorn 40 im Abstand umgebende Schraubenfeder 54 ab, deren anderes Ende am Boden des Aufnahmeschachtes 33 anliegt und somit das Außenteil 47 in Richtung auf die Druckgaspatrone 3 belastet. Dadurch wird auch das Innenteil 46 in derselben Richtung verschoben, da das Außenteil 47 über seine Innenfläche 49 an der Außenfläche 50 des Innenteils 46 anliegt. Die Schraubenfeder 54 schiebt damit das gesamte Dichtstück 45 gegen die Stirnfläche 41 der in den Anschlussschacht 33 eingesetzten Druckgaspatrone 3 und drückt dabei die Dichtfläche 48 gegen die Dichtkappe 35, so dass in diesem Bereich eine Abdichtung erfolgt.

Das Außenteil 47 des Dichtstückes 45 ist gegenüber der Innenwand des Anschlussschachtes 33 ebenfalls abgedichtet, und zwar einmal durch eine Ringdichtung 55, die in eine Umfangsnut 56 in der Innenwand des Anschlussschachtes 33 eingelegt ist und an der Außenfläche des Außenteiles 47 anliegt, und zum anderen durch eine Ringdichtung 57, die in eine Umfangsnut 58 in der Außenfläche des Außenteils 47 eingelegt ist und an der Innenwand des Anschlussschachtes 33 anliegt. Die Abdichtung in diesem Bereich könnte auch durch eine Ringdichtung erfolgen, die in eine Umfangsnut in der Innenwand des Anschlussschachtes 33 eingearbeitet ist, so dass die Ringdichtung dann dichtend an die Außenfläche des Außenteils 47 angelegt wäre, wie dies in Figur 12 dargestellt ist.

Durch die beschriebene Abdichtung wird das Dichtstück 45 gegenüber der Innenwand des Anschlussschachtes 33 abgedichtet, es ergibt sich damit ein abgedichteter Abschnitt 59 des Anschlussschachtes 33, der von der Druckgaspatrone 3 aus gesehen stromabwärts des Dichtstückes 45 angeordnet ist.

Dieses Dichtstück 45 steht vor dem Einschieben der Druckgaspatrone 3 in den Anschlussschacht 33 in einer Ruhestellung, bei der die Schraubenfeder 54 zumindest teilweise entspannt ist, dabei legt sich das hutförmige Innenteil 46 an einen Vorsprung 60 auf der Außenseite des Anstechdornes 40 an, der damit eine Verschiebung des Dichtstückes 45 begrenzt. Beim Einschieben einer Druckgaspatrone 3 schiebt diese dann das Dichtstück 45 aus dieser Ruhestellung entgegen der Wirkung der Schraubenfeder 54 in die Dichtstellung, in der die Schraubenfeder 54 das Dichtstück 45 gegen die Dichtkappe 35 der Druckgaspatrone 3 drückt.

Die von der Dichtung zwischen der Dichtkappe 35 und der Dichtfläche 48 eingeschlossene Querschnittsfläche des Dichtstückes 45 ist kleiner als die von den Ringdichtungen 55 und 57 eingeschlossene Querschnittsfläche, so dass aufgrund des Druckaufbaues in dem vom Dichtstück 45 abgeschlossenen Abschnitt 59 des Anschlussschachtes 33 eine Differenzkraft auf das Dichtstück 45 ausgeübt wird, welches die Wirkung der Schraubenfeder 54 verstärkt. Diese Differenzkraft nimmt mit zunehmendem Druck in dem Abschnitt 59 zu. Es handelt sich somit um eine selbstverstärkende Wirkung des Dichtstückes 45.

In der Innenwand des Anschlussschachtes 33 ist eine Umfangsnut 61 angeordnet, die außenseitig mit der Umgebung in Verbindung steht. Über diese Umfangsnut 61 kann also der Anschlussschacht 33 belüftet werden. Diese Umfangsnut 61 ist in Richtung auf das offene Ende des Anschlussschachtes 33 gegenüber der Ringdichtung 55 versetzt, so dass der Abschnitt 59 des Anschlussschachtes 33 gegenüber dieser Umfangsnut 61 abgedichtet ist, solange das Dichtstück 45 in der Dichtstellung steht. Dann liegt nämlich die Ringdichtung 55 an der Außenfläche des Dichtstückes 45 dichtend an.

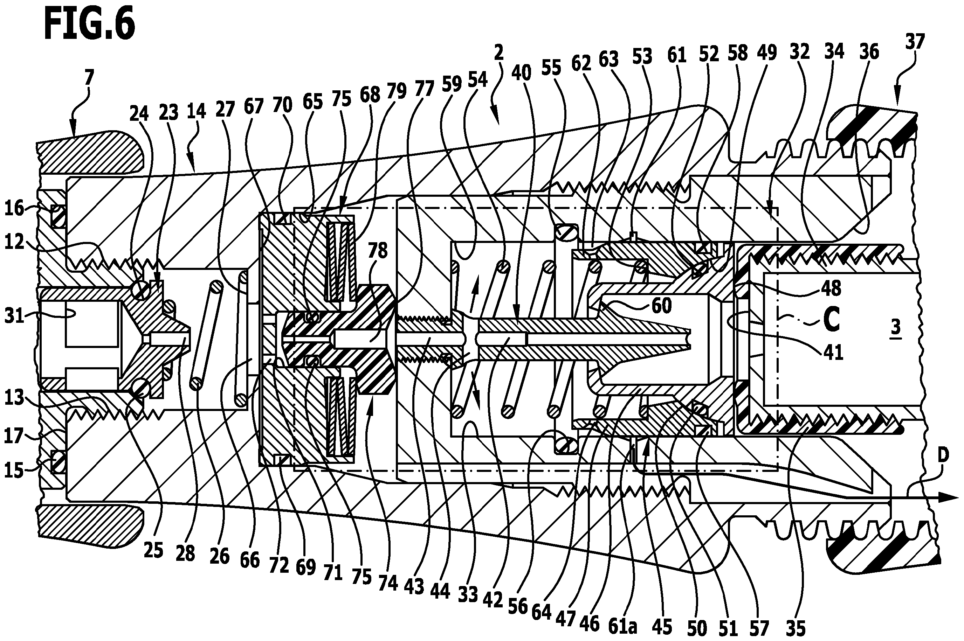

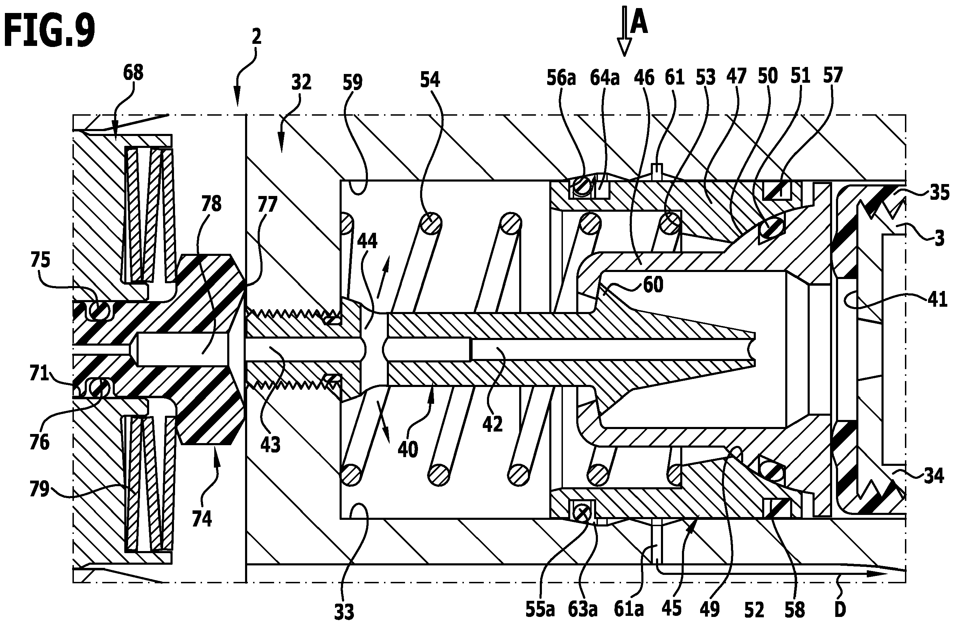

Die Umfangsnut 61 steht über eine Belüftungsbohrung 61a mit dem Innenraum in Verbindung, der einmal durch das Anschlussstück 14 und zum anderen durch die Überwurfhülse 37 umschlossen wird. Wie aus der Darstellung der Figur 12 deutlich wird, trägt die Überwurfhülse 37 an ihrem dem Innengewinde 39 abgewandten Ende mehrere Ausströmöffnungen 61b, durch die das durch die Umfangsnut 61 ausströmende Gas in die Umgebung abströmen kann. Das Ausströmen erfolgt dabei also in einem Bereich, der von den Funktionsteilen des Instrumentes maximal entfernt ist und bei dem keine Gefahr besteht, dass der Benutzer diesen Bereich ergreift, so dass auch keine Belästigung oder Gefährdung des Benutzers eintreten kann. Der Strömungsweg des über die Umfangsnut 61, die Belüftungsbohrung 61a, den Innenraum des Anschlussstückes 14 sowie der Überwurfhülse 37 und die Austrittsöffnungen 61b austretenden Gases ist in den Figuren 6 und 9 durch die Pfeile D angedeutet.

Die Belüftungsbohrung 61a hat einen sehr kleinen Querschnitt und wirkt somit als Drosselstelle zwischen der Umfangsnut 61 und dem Außenraum. Dadurch wird sichergestellt, dass die Strömungsgeschwindigkeit der austretenden Gasfüllung begrenzt wird, die Gasfüllung also nicht schlagartig entweicht.

Das Dichtstück 45 weist an seinem dem Boden des Anschlussschachtes 33 zugewandten Ende einen Randbereich 62 auf mit einem Außendurchmesser, der geringer ist als der Außendurchmesser der übrigen Außenfläche des Außenteils 47 des Dichtstückes 45. Sobald dieser Randbereich 62 in den Bereich der Ringdichtung 55 gelangt, wird daher die Dichtwirkung im Bereich der Ringdichtung 55 aufgehoben, das heißt es bildet sich an dieser Stelle ein schmaler Spalt 63 aus zwischen der Ringdichtung 55 und dem Randbereich 62 des Außenteils 47. Durch diesen Spalt 63 wird eine Verbindung zwischen dem Abschnitt 59 einerseits und der belüfteten Umfangsnut 61 andererseits hergestellt, und über diese Verbindung kann der Abschnitt 59 belüftet werden, das heißt ein in ihm aufgebauter Überdruck kann abgebaut werden.

Bei diesem Abbau können im Spalt 63 sehr hohe Strömungsgeschwindigkeiten entstehen, durch die die Gefahr besteht, dass die Ringdichtung 55 in den Spalt 63 hineingezogen und daher beschädigt wird, außerdem würde der Strömungspfad zwischen dem Abschnitt 59 und der Umfangsnut 61 dadurch verschlossen. Um dies zu verhindern, ist das hülsenförmige Außenteil 47 im Bereich des Randbereiches 62 mit radialen Entlastungsbohrungen 64 versehen, durch die Gas aus dem Abschnitt 59 in den Spalt 63 einströmen kann. Dadurch wird in diesem Bereich das Auftreten eines zu starken Unterdruckes verhindert und damit auch das ungewollte Einziehen der Ringdichtung 55 in den Spalt 63.

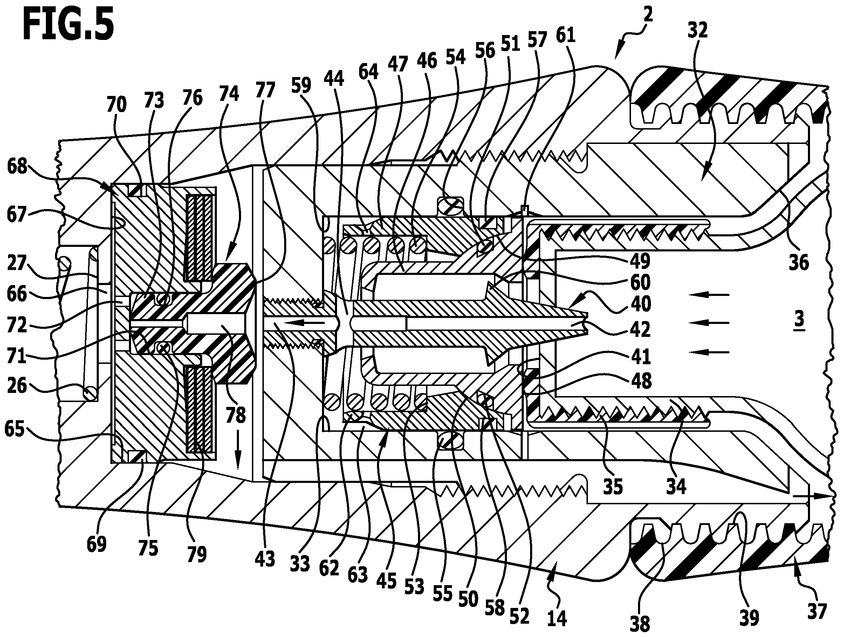

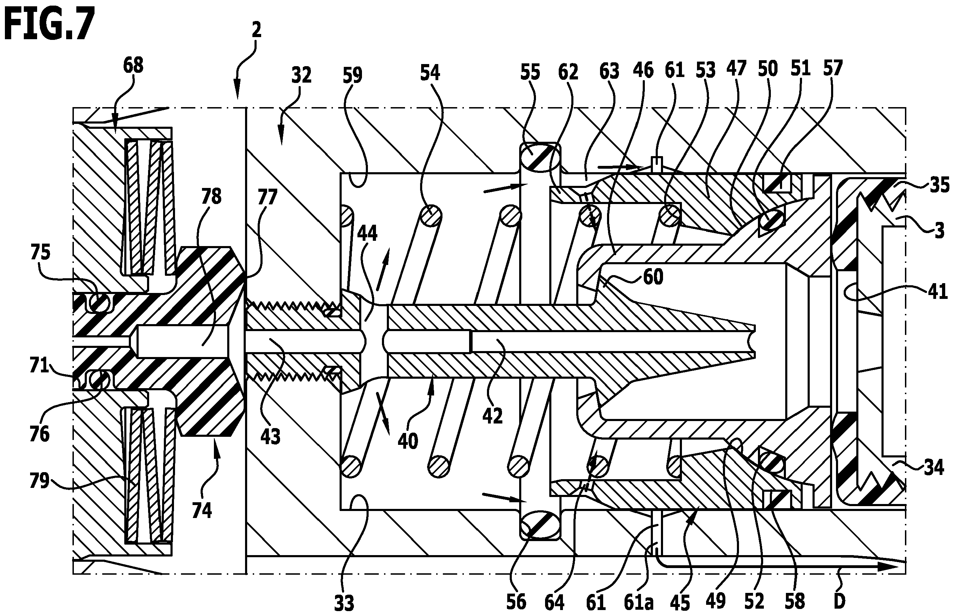

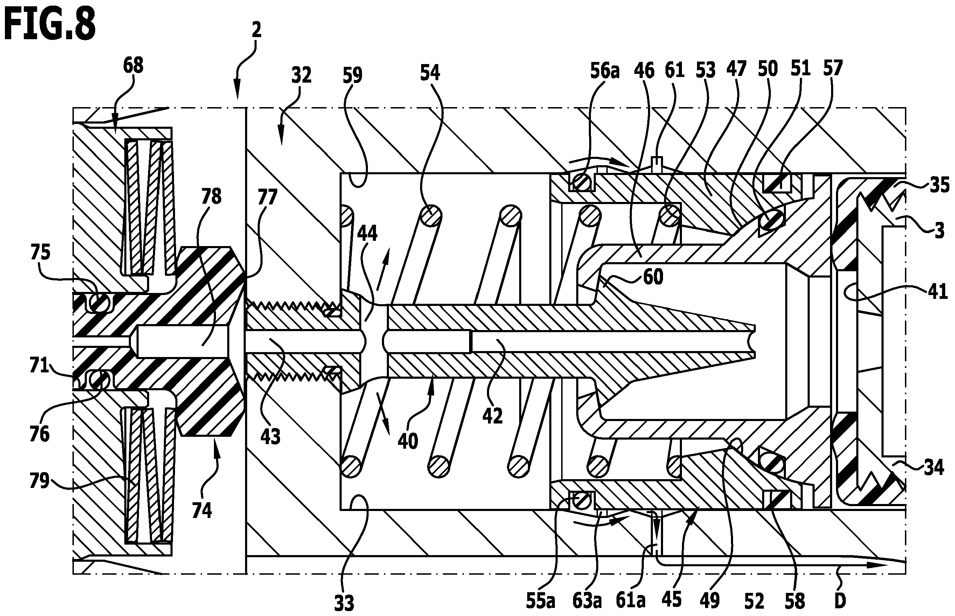

Während bei dem Ausführungsbeispiel der Figuren 1 bis 7 die Abdichtung zwischen Dichtstück 45 und der Innenwand des Anschlussschachtes 33 durch eine Ringdichtung 55 erfolgt, die in eine Umfangsnut 56 in der Innenwand des Anschlussschachtes 33 eingelegt ist, wird bei dem Ausführungsbeispiel der Figuren 8 bis 10, das im Übrigen weitgehend gleich aufgebaut ist und bei dem gleiche Teile daher mit denselben Bezugszeichen versehen sind, eine Abdichtung des Dichtstückes 45 gegenüber der Innenwand des Anschlussschachtes 33 durch eine Ringdichtung 55a erreicht, die in eine Umfangsnut 56a in der Außenwand des Dichtstückes 45 eingelegt ist und die sich normalerweise an die Innenwand des Anschlussschachtes 33 dichtend anlegt.

Bei dieser Ausgestaltung ist in der Innenwand des Anschlussschachtes 33 eine umfangsnutförmige Erweiterung 63a angeordnet, und zwar derart, dass die Ringdichtung 55a dieser Erweiterung 63a gegenüberliegt, wenn sich das Dichtstück 45 in der Belüftungsstellung befindet. Durch die Erweiterung 63a ergibt sich dadurch ein Strömungsweg, der an der Ringdichtung 55a vorbeiführt und den stromabwärts des Dichtstückes 45 gelegenen Abschnitt 59 des Anschlussschachtes 33 verbindet mit der Umfangsnut 61 und der Belüftungsbohrung 61a. Dieses Auftreten eines Nebenstrompfades entspricht bei dem Ausführungsbeispiel der Figuren 1 bis 7 der Belüftungsstellung, bei der der Randbereich 62 mit dem kleineren Außendurchmesser der Ringdichtung 55 gegenübersteht.

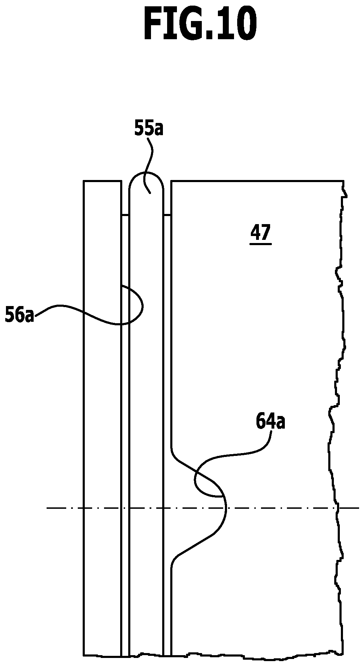

Bei dem in Figur 9 dargestellten Ausführungsbeispiel könnte die Ringdichtung 55a durch die an ihr vorbeiströmende Gasströmung mitgenommen und so aufgeweitet werden, dass sie nach außen in die Erweiterung 63a hineingedrückt wird und somit den Spalt zwischen dem Dichtstück 45 und der Innenwand des Anschlussschachtes 33 verschließt. Dann wäre eine Strömungsverbindung zu der Umfangsnut 61 und der Belüftungsbohrung 61a unterbunden. Um dies zu verhindern, ist bei dem Ausführungsbeispiel der Figuren 9 und 10 seitlich an der Umfangsnut 56a mindestens eine Ausnehmung 64a angeordnet, die im Wesentlichen als Vertiefung der Seitenwand der Umfangsnut 56a ausgebildet ist und die sicherstellt, dass auch bei aufgeweiteter Ringdichtung 55a die Umfangsnut 56a mit der Umfangsnut 61 in Strömungsverbindung steht, wenn das Dichtstück 45 in der Belüftungsstellung steht. Auch wenn sich die Ringdichtung 55a aufweitet, kann sie nämlich die Ausnehmung 64a oder die Ausnehmungen 64a, die über den Umfang der Umfangsnut 56a verteilt sein können, nicht verschließen, so dass in diesem Bereich ein Strömungsweg frei bleibt.

Solange die Überwurfhülse 37 fest auf das Anschlussstück 14 aufgeschraubt ist und dadurch die Druckgaspatrone 3 vollständig in den Anschlussschacht 33 eingeschoben ist, bleibt das Dichtstück 45 in der Dichtstellung, die Umfangsnut 61 bleibt daher verschlossen. Sobald der Benutzer jedoch die Überwurfhülse 37 teilweise vom Anschlussstück 14 abschraubt, wird das Dichtstück 45 unter dem Einfluss der Schraubenfeder 54 und der vom Gasdruck hervorgerufenen Druckdifferenz in Richtung auf die Ruhestellung verschoben, da die Druckgaspatrone 3 nunmehr frei ist, zumindest teilweise aus dem Anschlussschacht 33 auszutreten. Dabei gelangt das Dichtstück zwischen der Dichtstellung und der Ruhestellung in eine dazwischen liegende Entlastungsstellung, in der der Randbereich 62 der Ringdichtung 55 gegenüberliegt und in der sich die Umfangsnut 61 zum Abschnitt 59 hin öffnet. Dies führt dazu, dass das Druckgas aus der Druckgaspatrone in die Umgebung entweichen kann, allerdings nicht schlagartig, sondern allmählich, da der Querschnitt der Umfangsnut 61 und der sich anschließenden Bohrung 61a klein ist. Es erfolgt also beim Abschrauben der Überwurfhülse 37 gleichzeitig mit diesem Abschraubvorgang eine Entleerung der Druckgaspatrone 3 ohne ein plötzliches Ausströmen des Druckgases und ohne das damit verbundene unangenehme Zischen des Druckgases. Durch die Abdichtung zwischen dem Dichtstück 45 und der Innenwand des Anschlussschachtes 33 durch die Ringdichtung 57 wird auch sichergestellt, dass die ausströmende Gasfüllung ausschließlich über die Umfangsnut 61 strömt und nicht unkontrolliert zwischen dem Dichtstück 45 und der Innenwand des Anschlussschachtes 33 austritt.

Ein vollständiges Abschrauben der Überwurfhülse 37 und damit eine vollständige Entnahme der Druckgaspatrone 3 ist erst nach einigen Umdrehungen der Überwurfhülse 37 möglich, und diese Zeit reicht aus, um die Druckgaspatrone 3 zumindest weitgehend zu entleeren, so dass bei der Entnahme der Druckgaspatrone 3 aus dem Anschlussschacht 33 diese ganz oder weitgehend entleert ist.

Zwischen dem Boden des Anschlussschachtes 33 einerseits und zwischen der Stufe 27 des Anschlussstückes 14 andererseits befindet sich im Anschlussstück 14 eine Ventilkammer 65, in die einerseits der Kanal 42 des Anstechdornes 40 einmündet und andererseits eine von der Stufe 27 umgebene Austrittsöffnung 66 austritt. Diese Austrittsöffnung 66 hat einen wesentlich kleineren Durchmesser als die Ventilkammer 65, so dass im Übergang von der Ventilkammer 65 zu der Austrittsöffnung 66 eine die Austrittsöffnung 66 umgebende Stufe 67 ausgebildet wird.

Im Inneren der Ventilkammer 65 ist ein scheibenförmiger Ventilkörper 68 verschieblich gelagert, der sich mittels einer ersten Ringdichtung 69, die in eine Umfangsnut 70 in der Außenwand des Ventilkörpers 68 eingelegt ist, gegenüber der Innenwand der Ventilkammer 65 abdichtet. In den Ventilkörper 68 ist auf dessen dem Aufnahmekörper 32 zugewandter Seite eine zentrale Lagerkammer 71 angeordnet in Form einer zylindrischen Sacklochbohrung, die an ihrem zentral verschlossenen Ende durch außermittig angeordnete Strömungsdurchlässe 72 mit dem stromabwärts des Ventilkörpers 68 angeordneten Abschnitt der Ventilkammer 65 und damit mit der Austrittsöffnung 66 verbunden ist.

In diese zylindrische Lagerkammer 71 ist der zylindrische Lagerstutzen 73 eines Anschlusselementes 74 eingeschoben und längsverschieblich in dieser Lagerkammer 71 gelagert. Eine zweite Ringdichtung 75, die in eine Umfangsnut 76 des Lagerstutzens 73 eingelegt ist, dichtet den Lagerstutzen 73 gegenüber der Innenwand der Lagerkammer 71 ab.

Das Anschlusselement 74 verbreitert sich außerhalb der Lagerkammer 71 und bildet in diesem Bereich eine ringförmige Dichtschulter 77 aus, die normalerweise dichtend an die Rückseite des Bodens des Aufnahmekörpers 32 angelegt ist und den Austritt des Anstechdorns 40 in die Ventilkammer 65 abdichtend umgibt. Das Anschlusselement 74 kann aus Kunststoffmaterial bestehen und insbesondere im Bereich der Dichtschulter 77 verformbar ausgebildet sein. Es wird von einem Strömungskanal 78 durchsetzt, der damit einerseits den Kanal 42 im Inneren des Anstechdorns 40 und andererseits die Lagerkammer 71 in dem unmittelbar an die Strömungsdurchlässe 72 anschließenden Bereich miteinander verbindet.

Der Durchmesser D der Ventilkammer 65 und des Ventilkörpers 68 ist im Wesentlichen gleich groß, während der Durchmesser d der Dichtschulter 77 des Anschlusselementes 74 demgegenüber deutlich kleiner ist (Figur 2), beispielsweise gilt D=19mm und d=6mm, so dass das Verhältnis der wirksamen druckbeaufschlagten Flächen des Ventilkörpers 68 und des Anschlusselementes 74 bei etwa 10 liegt. Diese Verhältnisse können abweichen, beispielsweise kann das Verhältnis d:D zwischen 1:2 und 1:4 liegen.

An der dem Aufnahmekörper 32 zugewandten Seite stützt sich an dem Ventilkörper 68 eine das Anschlusselement 74 umgebende Tellerfeder 79 ab, die sich auf ihrer gegenüberliegenden Seite an dem verbreiterten Teil des Anschlusselementes 74 abstützt. Diese Tellerfeder 79 drückt damit den Ventilkörper 68 und das Anschlusselement 74 auseinander und drückt normalerweise den Ventilkörper 68 gegen die Stufe 67 und die Dichtschulter 77 des Anschlusselementes 74 gegen die Rückseite des Bodens des Anschlussschachtes 33 (Figur 3). In dieser Stellung kann durch den Anstechdorn 40 aus der Druckgaspatrone 3 austretendes Gas ungehindert durch das Anschlusselement 74 und den Ventilkörper 68 über die Strömungsdurchlässe 72 und die Austrittsöffnung 66 sowie den geöffneten Schließkörper 23 in den Anschlussstutzen 1 strömen. Der Schließkörper 23 steht dabei in seiner Öffnungsstellung, das Anschlusselement 74 in seiner Dichtstellung.

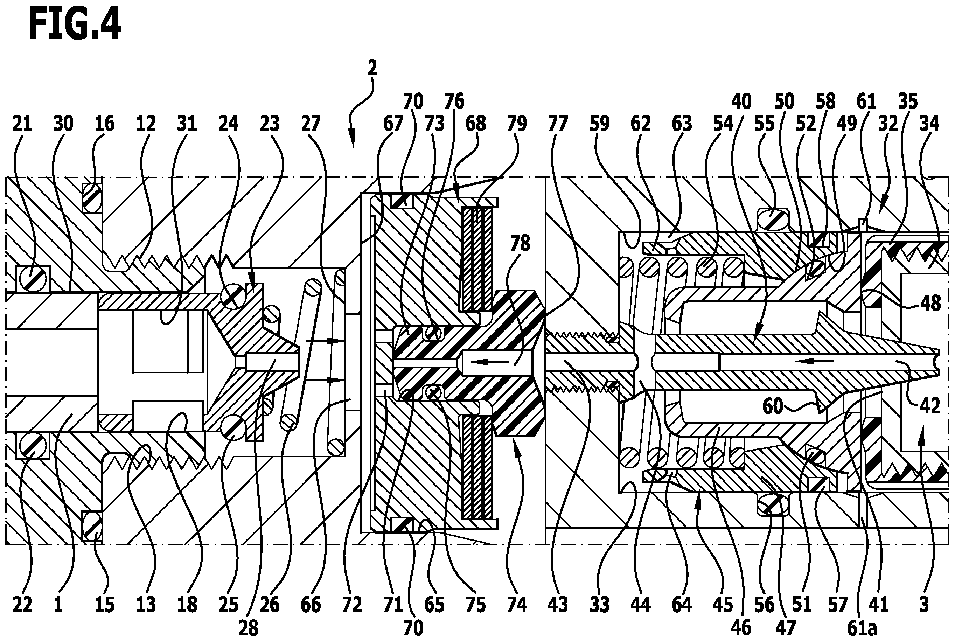

Wenn sich der Druck des ausströmenden Gases in der Lagerkammer 65 stromabwärts der ersten Ringdichtung 69 über einen bestimmten Wert erhöht, führt dies dazu, dass auf den Ventilkörper 68 eine Kraft wirkt, die der Federkraft der Tellerfeder 79 entgegengesetzt ist, da der stromaufwärts der ersten Ringdichtung 69 angeordnete Teil der Ventilkammer 65 nicht von dem Druckgas beaufschlagt ist. Dies führt beim Überschreiten eines bestimmten Gasdruckes dazu, dass der Ventilkörper 68 entgegen der Wirkung der Tellerfeder 70 gegen das Anschlusselement 74 verschoben wird, bis der verschlossene zentrale Bereich der Lagerkammer 71 sich abdichtend an die Stirnseite des Anschlusselementes 74 anlegt und dadurch den Strömungskanal 78 verschließt (Figur 4). Damit ist ein weiteres Nachströmen von Gas aus der Druckgaspatrone 3 beendet, eine Öffnung des Ventilkörpers 68 ergibt sich erst wieder, wenn der stromabwärts des Ventilkörpers 68 herrschende Druck abgebaut ist. Auf diese Weise wird der maximale Druck begrenzt, der in dem Bereich stromabwärts des Ventilkörpers 68 herrscht. Der Ventilkörper 68 wirkt als Druckbegrenzungsventil.

Im normalen Betrieb bleibt das Anschlusselement 74 immer in der abdichtenden Anlage am Aufnahmekörper 32. Wenn jedoch der Druck in der Druckgaspatrone 3 den normalen Maximaldruck des Gases in der Druckgaspatrone überschreitet, beispielsweise bei einem Sterilisiervorgang und der damit verbundenen Temperaturerhöhung, kann die Kraft, die von dem unter Druck stehenden Gas auf das Anschlusselement 74 ausgeübt wird und das Anschlusselement 74 in Strömungsrichtung zu verschieben sucht, so groß werden, dass entgegen der Wirkung der Tellerfeder 79 das Anschlusselement 74 von der Rückseite des Bodens des Anschlussschachtes 33 abgehoben wird, so dass nunmehr in den stromaufwärts der ersten Ringdichtung 69 gelegenen Abschnitt der Ventilkammer 65 Gas eintreten kann, das heißt es erfolgt eine Belüftung in diesem Bereich und ein Druckabbau des in der Druckgaspatrone 3 und dem anschließenden Strömungskanal herrschenden Überdruckes. Damit wirkt das Anschlusselement 74 als Sicherheitsventil, das beim Überschreiten des Maximalwertes des Druckes, der deutlich über dem normalen Betriebsdruck liegt, öffnet und diesen Überdruck abbaut.

Bei einem Sterilisiervorgang ist normalerweise der Anschluss 2 vom Anschlussstutzen 1 getrennt, so dass der Schließkörper 23 in Schließstellung steht. Dadurch herrscht stromabwärts des Ventilkörpers 68 normalerweise derselbe Druck wie stromaufwärts. Aufgrund der unterschiedlichen Querschnitte des Ventilkörpers 68 einerseits und des Anschlusselementes 74 andererseits führt dies dazu, dass das Anschlusselement 74 abdichtend am Aufnahmekörper 32 anliegt.

Bei einer Temperaturerhöhung, beispielsweise während eines Sterilisiervorgangs, steigt der Druck stromabwärts des Ventilkörpers 68 entsprechend der normalen Temperaturabhängigkeit des Druckes bei Gasen proportional zur Zunahme der Temperatur an, während auf der stromaufwärts gelegenen Seite der Druck überproportional ansteigt. Dies liegt darin, dass das Druckgas in der Druckgaspatrone in flüssiger Form vorliegt und beim Temperaturanstieg in einen überkritischen Bereich gelangt, in dem der Druck bei Temperaturerhöhung sehr viel stärker ansteigt als bei einem reinen Gas. Dieser Druckanstieg auf der stromaufwärts gelegenen Seite des Ventilkörpers und des Anschlusselementes führt schließlich zu einem Abheben des Anschlusselementes 74 von dem Aufnahmekörper 32, wenn das Verhältnis des Drucks stromaufwärts des Anschlusselementes 74 zum Druck stromabwärts des Ventilkörpers 68 größer wird als das Flächenverhältnis, das sich durch die unterschiedlichen Querschnitte des Ventilkörpers 68 einerseits (Durchmesser D) und des Anschlusselementes 74 andererseits (Durchmesser d) ergibt.

Sowohl der Ventilkörper 68 als auch das Anschlusselement 74 werden dabei durch dieselbe Tellerfeder 79 beaufschlagt, die einmal dafür sorgt, dass normalerweise der Ventilkörper 68 in der Öffnungsstellung steht und das Anschlusselement 74 in der Dichtstellung, die Differenz zwischen der Kraft der Tellerfeder 79 einerseits und der jeweils auf den Ventilkörper 68 beziehungsweise das Anschlusselement 74 wirkenden Überdruckkraft wird dann zur Verstellung des Ventilkörpers 68 beziehungsweise des Anschlusselementes 74 verwendet.

Trotz der beschriebenen Abdichtungsmaßnahmen ist nicht ganz auszuschließen, dass im Anschlussbereich der Druckgaspatrone 3 bei der Dichtung der Dichtkappe 35 und der Dichtfläche 48 unerwünscht Gas seitlich in den Anschlussschacht 33 austreten könnte. Um einen Aufbau des Gases in diesem Bereich zu verhindern, ist es günstig, wenn die Dichtkappe 35 in ihrem seitlichen Randbereich kanalförmige Vertiefungen 80 aufweist, durch die das Gas seitlich an der Dichtkappe 35 vorbei aus dem Teil des Anschlussschachtes 33 nach außen abströmen kann, der durch das Dichtstück 45 gegenüber dem Abschnitt 59 des Anschlussschachtes 33 abgedichtet ist.

## Patentansprüche

1. Druckgasbetriebenes Instrument mit einem Anschluss (2) für eine Druckgaspatrone (3), an dem die Druckgaspatrone (3) abgedichtet mit einem Zufuhrkanal (42) für aus der Druckgaspatrone (3) ausströmendes Druckgas verbindbar ist, und mit einem Regelventil in dem Zufuhrkanal (42), welches den Druckgasstrom durch den Zufuhrkanal (42) beeinflusst, wobei das Regelventil einen in einer Ventilkammer (65), durch die der Zufuhrkanal (42) hindurchführt, gegenüber der Wand der Ventilkammer (65) mittels einer ersten Dichtung (69) abgedichteten, in der Ventilkammer (65) verschieblich gelagerten Ventilkörper (68) umfasst, wobei der Ventilkörper (68) auf seiner stromaufwärts gelegenen Seite mit einem Anschlusselement (74) mittels einer zweiten Dichtung (75) abgedichtet verbunden und bei einer Verschiebung in der Ventilkammer (65) auch gegenüber dem Anschlusselement (74) verschiebbar ist, wobei das Anschlusselement (74) einen durchgehenden Strömungskanal (78) aufweist und einströmseitig mit dem Zufuhrkanal (42) abgedichtet verbunden ist, wobei der Ventilkörper (68) einen Strömungsdurchlass (72) aufweist, der ausströmseitig mit dem stromabwärts an die Ventilkammer (65) anschließenden Zufuhrkanal (66, 18) in Verbindung steht, wobei der Ventilkörper (68) in der Ventilkammer (65) zwischen einer Öffnungsstellung, in der eine Strömungsverbindung zwischen dem Strömungskanal (78) des Anschlusselements (74) und dem Strömungsdurchlass (72) des Ventilkörpers (68) besteht, und einer Schließstellung entgegen der Strömungsrichtung verschiebbar ist, in der der Ventilkörper (68) sich abdichtend an den Strömungskanal (78) des Anschlusselements (74) anlegt und diesen dadurch verschließt, wobei der Ventilkörper (68) durch ein Federelement (79) in Richtung auf die Öffnungsstellung belastet ist, wobei die von der ersten Dichtung (69) umschlossene Querschnittsfläche des Ventilkörpers (68) größer ist als die von der zweiten Dichtung (75) umschlossene Querschnittsfläche, wobei stromabwärts des Regelventils (68, 74) im Zufuhrkanal (18) ein Schließkörper (23) verschieblich gelagert ist, der von einem von der stromabwärts gelegenen Seite in den Zufuhrkanal (18) eingeschobenen Anschlussstutzen (1) von einer stromabwärts gelegenen Schließposition in eine stromaufwärts gelegene Öffnungsposition verschiebbar ist, wobei der Schließkörper (23) in der Öffnungsposition den Durchgang durch den Strömungskanal (18) freigibt und ihn in der Schließposition verschließt, und wobei der Schließkörper (23) einen Durchlass (28) aufweist, der auch bei der Stellung des Schließkörpers (23) in der Schließposition eine sehr reduzierte Strömung des Druckgases an dem Schließkörper (23) vorbei zulässt.

2. Instrument nach Anspruch 1, **dadurch gekennzeichnet, dass** das Federelement (79) eine Tellerfeder ist.

3. Instrument nach einem der Ansprüche 1 oder 2, **dadurch gekennzeichnet, dass** der Ventilkörper (68) eine zentrale, zur Einströmseite hin offene Lagerkammer (71) aufweist, in die ein Lagerstutzen (73) des Anschlusselementes (74) mittels der zweiten Dichtung (75) abgedichtet und verschiebbar eintaucht, und dass aus der Lagerkammer (71) an deren ausströmseitigem Ende der Strömungsdurchlass (72) austritt.

4. Instrument nach einem der voranstehenden Ansprüche, **dadurch gekennzeichnet, dass** der Ventilkörper (68) in der Schließstellung an der ausströmseitigen Stirnseite des Anschlusselementes (74) anliegt und dadurch den Strömungskanal (78) verschließt.

5. Instrument nach einem der voranstehenden Ansprüche, **dadurch gekennzeichnet, dass** das Anschlusselement (74) durch ein Federelement (79) und/oder durch den Druck stromabwärts des Ventilkörpers (68) in eine Dichtstellung gedrückt wird, in der das Anschlusselement (74) dicht an dem in die Ventilkammer (65) eintretenden Zufuhrkanal (42) anliegt und diesen dadurch dicht mit dem Strömungskanal (78) im Anschlusselement (74) verbindet, und gegen die Wirkung des Federelements (79) von dem Zufuhrkanal (42) in eine Entlastungsstellung verschiebbar ist, in der der in die Ventilkammer (65) eintretende Zufuhrkanal (42) in den stromaufwärts der ersten Dichtung (69) gelegenen Teil der Ventilkammer (65) geöffnet ist.

6. Instrument nach Anspruch 5, **dadurch gekennzeichnet, dass** das den Ventilkörper (68) in die Öffnungsstellung und das Anschlusselement (74) in die Dichtstellung verschiebende Federelement (79) dasselbe Federelement ist.

7. Instrument nach einem der Ansprüche 5 oder 6, **dadurch gekennzeichnet, dass** das Verhältnis der stromabwärts gelegenen Querschnittsfläche des Ventilkörpers (68) zu der stromaufwärts gelegenen Querschnittsfläche des Anschlusselements (74) größer ist als das Verhältnis des Druckes in der Druckgaspatrone zu dem geregelten Druck stromabwärts des Ventilkörpers (68) bei Raumtemperatur und kleiner ist als das Verhältnis diese Drücke bei gegenüber Raumtemperatur deutlich erhöhter Temperatur.

8. Instrument nach einem der voranstehenden Ansprüche, **dadurch gekennzeichnet, dass** der Durchlass (28) ein durch den Schließkörper (23) hindurchgehender Kanal mit geringem Querschnitt ist.

9. Instrument nach einem der voranstehenden Ansprüche, **dadurch gekennzeichnet, dass** der Durchlass durch einen porösen Wandbereich des Schließkörpers gebildet wird.

10. Instrument nach einem der Ansprüche 1 bis 7, **dadurch gekennzeichnet, dass** der Schließkörper (23) durch eine Feder (26) in Richtung auf die Schließposition belastet ist.

11. Instrument nach einem der Ansprüche 1 bis 8, **dadurch gekennzeichnet, dass** der Schließkörper (23) in der Schließposition durch eine Dichtung (25) gegenüber dem Zufuhrkanal (18) abgedichtet ist.

## Claims

1. A compressed gas operated instrument comprising a connector (2) for a compressed gas cartridge (3) to which the compressed gas cartridge (3) is connectable in sealed manner by a supply channel (42) for compressed gas flowing out of the compressed gas cartridge (3), and also comprising a regulating valve in the supply channel (42) which governs the stream of compressed gas flowing through the supply channel (42), wherein the regulating valve comprises a valve body (68) which is mounted in a valve chamber (65) through which the supply channel (42) extends, said valve body (68) being displaceable in the valve chamber (65) and sealed with respect to the wall of the valve chamber (65) by means of a first seal (69), wherein the valve body (68) is connected on the upstream side thereof in sealed manner to a connector element (74) by means of a second seal (75) and is also displaceable with respect to the connector element (74) by displacement thereof in the valve chamber (65), wherein the connector element (74) comprises a continuous flow channel (78) and is connected at the inlet end thereof to the supply channel (42) in sealed manner, wherein the valve body (68) comprises a flow passage (72) which is connected at the outlet end thereof to the supply channel (66, 18) located downstream of the valve chamber (65), wherein the valve body (68) is displaceable in the valve chamber (65) against the direction of flow between an open position in which there is a flowing connection between the flow channel (78) of the connector element (74) and the flow passage (72) of the valve body (68) and a closed position in which the valve body (68) abuts in sealed manner against the flow channel (78) of the connector element (74) thereby closing it, wherein the valve body (68) is biased in the direction of the open position by a spring element (79), wherein the cross-sectional area of the valve body (68) surrounded by the first seal (69) is larger than the cross-sectional area surrounded by the second seal (75), wherein a closure body (23) is mounted in the supply channel (18) downstream of the regulating valve (68, 74) in displaceable manner, said closure body being displaceable by a connector fitting (1) that is inserted into the supply channel (18) from the downstream side thereof from a closed position located downstream into an open position located upstream, wherein the closure body (23) unblocks the passage through the flow channel (18) in the open position and blocks it in the closed position, and wherein the closure body (23) comprises a passage (28) which permits a very much reduced current of the compressed gas to flow past the closure body (23) even when the closure body (23) is positioned in the closed position.

2. An instrument in accordance with Claim 1, **characterized in that** the spring element (79) is a disc spring.

3. An instrument in accordance with any of the Claims 1 or 2, **characterized in that** the valve body (68) comprises a central bearing chamber (71) which is open at the gas inlet end and into which there projects a displaceable bearing stem (73) of the connector element (74) that is sealed by means of the second seal (75), and **in that** the flow passage (72) emerges from the bearing chamber (71) at the gas outlet end thereof.

4. An instrument in accordance with any of the preceding Claims, **characterized in that**, in the closed position thereof, the valve body (68) abuts the gas outlet end face of the connector element (74) and thereby closes the flow channel (78).

5. An instrument in accordance with any of the preceding Claims, **characterized in that** the connector element (74) is pressed by a spring element (79) and/or by the pressure downstream of the valve body (68) into a sealing position in which the connector element (74) tightly abuts against the supply channel (42) entering the valve chamber (65) and thereby connects said supply channel in gas-tight manner to the flow channel (78) in the connector element (74), and is displaceable against the effect of the spring element (79) from the supply channel (42) into a disengaged position in which the supply channel (42) entering the valve chamber (65) is open into the part of the valve chamber (65) that is located upstream of the first seal (69).

6. An instrument in accordance with Claim 5, **characterized in that** the spring element (79) displacing the valve body (68) into the open position and the connector element (74) into the sealing position is the same spring element.

7. An instrument in accordance with any of the Claims 5 or 6, **characterized in that** the ratio of the cross-sectional area of the valve body located downstream (68) to the cross-sectional area of the connector element (74) located upstream is larger than the ratio of the pressure in the compressed gas cartridge to the regulated pressure downstream of the valve body (68) at ambient temperature and is smaller than the ratio of these pressures at temperatures that are significantly higher compared to the ambient temperature.

8. An instrument in accordance with any of the preceding Claims, **characterized in that** the passage (28) is a channel of small cross section passing through the closure body (23).

9. An instrument in accordance with any of the preceding Claims, **characterized in that** the passage is formed by a porous wall region of the closure body.

10. An instrument in accordance with any of the Claims 1 to 7, **characterized in that** the closure body (23) is biased in the direction of the closed position by a spring (26).

11. An instrument in accordance with any of the Claims 1 to 8, **characterized in that**, in the closed position thereof, the closure body (23) is sealed with respect to the supply channel (18) by a seal (25).

## Revendications

1. Instrument fonctionnant au gaz comprimé comprenant un raccord de branchement (2) pour une cartouche de gaz comprimé (3), au niveau duquel la cartouche de gaz comprimé (3) peut être reliée de manière étanche à un canal d'alimentation (42) pour le gaz comprimé s'écoulant hors de la cartouche de gaz comprimé (3), et comprenant une valve de régulation dans le canal d'alimentation (42), qui influence l'écoulement de gaz comprimé à travers le canal d'alimentation (42), instrument
dans lequel la valve de régulation comprend dans une chambre de valve (65), à travers laquelle mène le canal d'alimentation (42), un corps de valve (68), qui est monté de manière coulissante dans la chambre de valve (65), et est rendu étanche par rapport à la paroi de la chambre de valve (65) au moyen d'un premier joint d'étanchéité (69),
dans lequel le corps de valve (68) est, sur son côté amont, relié de manière étanche à un élément de raccordement (74) au moyen d'un deuxième joint d'étanchéité (75), et peut lors d'un coulissement dans la chambre de valve (65), également coulisser par rapport à l'élément de raccordement (74),
dans lequel l'élément de raccordement (74) présente un canal d'écoulement (78) traversant, et est relié de manière étanche, du côté de l'entrée d'écoulement, au canal d'alimentation (42),
dans lequel le corps de valve (68) présente un passage d'écoulement (72) qui, du côté de la sortie d'écoulement, est en liaison avec le canal d'alimentation (66, 18) se raccordant à la chambre de valve (65) du côté aval,
dans lequel le corps de valve (68) peut coulisser dans la chambre de valve (65) à l'encontre de la direction d'écoulement, entre une position d'ouverture dans laquelle est établie une liaison d'écoulement entre le canal d'écoulement (78) de l'élément de raccordement (74) et le passage d'écoulement (72) du corps de valve (68), et une position de fermeture dans laquelle le corps de valve (68) s'applique de manière étanche contre le canal d'écoulement (78) de l'élément de raccordement (74) en produisant ainsi son obturation,
dans lequel le corps de valve (68) est chargé par un élément de ressort (79), en direction de la position d'ouverture,
dans lequel la surface de section transversale du corps de valve (68), qui est entourée par le premier joint d'étanchéité (69) est supérieure à la surface de section transversale entourée par le deuxième joint d'étanchéité (75),
dans lequel aval de la valve de régulation (68, 74), dans le canal d'alimentation (18), est monté coulissant un corps d'obturation (23), qui peut coulisser sous l'effet d'un embout de raccordement (1) inséré dans le canal d'alimentation (18) à partir du côté situé en aval, d'une position de fermeture située en aval à une position d'ouverture située en amont,
dans lequel le corps d'obturation (23), dans la position d'ouverture, libère le passage à travers le canal d'écoulement (18), et le ferme dans la position de fermeture,
et dans lequel le corps d'obturation (23) présente un passage (28) qui, même dans le cas du positionnement du corps d'obturation (23) dans la position de fermeture, autorise un écoulement très réduit du gaz comprimé par-delà le corps d'obturation (23).

2. Instrument selon la revendication 1, **caractérisé en ce que** l'élément de ressort (79) est une rondelle-ressort.

3. Instrument selon l'une des revendications 1 ou 2, **caractérisé en ce que** le corps de valve (68) présente une chambre de montage (71) centrale, ouverte en direction du côté de l'entrée d'écoulement, et dans laquelle s'engage un embout de montage (73) de l'élément de raccordement (74), de manière coulissante et étanche au moyen du deuxième joint d'étanchéité (75), et **en ce que** le passage d'écoulement (72) sort de la chambre de montage (71), à son extrémité du côté sortie d'écoulement.

4. Instrument selon l'une des revendications précédentes, **caractérisé en ce que** dans la position de fermeture, le corps de valve (68) s'appuie contre le côté frontal de sortie d'écoulement de l'élément de raccordement (74), et ferme ainsi le canal d'écoulement (78) .

5. Instrument selon l'une des revendications précédentes, **caractérisé en ce que** l'élément de raccordement (74) est pressé par un élément de ressort (79) et/ou par la pression en aval du corps de valve (68), dans une position d'étanchéité dans laquelle l'élément de raccordement (74) s'appuie de manière étanche contre le canal d'alimentation (42) pénétrant dans la chambre de valve (65) en reliant ainsi celui-ci de manière étanche au canal d'écoulement (78) dans l'élément de raccordement (74), et peut coulisser à l'encontre de l'action de l'élément de ressort (79), à partir du canal d'alimentation (42), dans une position de décharge dans laquelle le canal d'alimentation (42) pénétrant dans la chambre de valve (65) est ouvert dans la partie de la chambre de valve (65) située en amont du premier joint d'étanchéité (69).

6. Instrument selon la revendication 5, **caractérisé en ce que** l'élément de ressort (79) faisant coulisser le corps de valve (68) dans la position d'ouverture et l'élément de raccordement (74) dans la position d'étanchéité, est le même élément de ressort.

7. Instrument selon l'une des revendications 5 ou 6, **caractérisé en ce que** le rapport de la surface de section transversale du corps de valve (68), située en aval, à la surface de section transversale de l'élément de raccordement (74), située en amont, est supérieur au rapport de la pression dans la cartouche de gaz comprimé à la pression régulée en aval du corps de valve (68) à température ambiante, et est inférieur au rapport de ces pressions à une température nettement plus élevée que la température ambiante.

8. Instrument selon l'une des revendications précédentes, **caractérisé en ce que** le passage (28) est un canal de faible section transversale traversant le corps d'obturation (23).

9. Instrument selon l'une des revendications précédentes, **caractérisé en ce que** le passage est formé par une zone de paroi poreuse du corps d'obturation.

10. Instrument selon l'une des revendications 1 à 7, **caractérisé en ce que** le corps d'obturation (23) est chargé par un ressort (26) en direction de la position de fermeture.

11. Instrument selon l'une des revendications 1 à 8, **caractérisé en ce que** dans la position de fermeture, le corps d'obturation (23) est rendu étanche par rapport au canal d'alimentation (18), par un joint d'étanchéité (25) .
